# EUROPEAN PATENT APPLICATION

(11) **EP 3 795 178 A1**
(43) Date of publication of application: **24.03.2021**
(21) Application number: 19306138.9
(22) Date of filing: 19.09.2019
(51) Int. Cl.: A61K 41/00, A61K 47/69, A61K 49/18, A61P 35/00, A61P 1/16, A61P 9/10, A61P 11/00, A61P 37/08, A61K 39/00, A61N 5/10

(54) **METHODS FOR TRIGGERING M1 MACROPHAGE POLARIZATION**

(71) Applicant: NH THERAGUIX, 38240 Meylan (FR); Institut Gustave Roussy, 94805 Villejuif Cédex (FR); UNIVERSITE CLAUDE BERNARD - LYON 1, 69100 Villeurbanne (FR); CENTRE NATIONAL DE LA RECHERCHE SCIENTIFIQUE -CNRS-, 75016 Paris (FR); INSERM (Institut National de la Santé et de la Recherche Médicale), 75013 Paris (FR); Universite Paris-Sud, 91400 Orsay (FR)
(72) Inventor: ALLOUCH, Awatef, 94360 BRY SUR MARNE (FR); DEUTSCH, Eric, 75011 PARIS (FR); PERFETTINI, Jean-Luc, 77100 MEAUX (FR); LUX, François, 69003 LYON (FR); TILLEMENT, Olivier, 69270 FONTAINES SAINT-MARTIN (FR)
(74) Representative: Plasseraud IP

(57) **Abstract**

The present disclosure relates to the field of nanomedicine, in particular for treating cancers. The present disclosure more specifically provides new methods of treating undesirable M2-polarized macrophages and/or inducing M1 macrophage polarization in a subject in need thereof, comprising administering to said subject a therapeutically effective amount of nanoparticles containing metallic elements.

## Description

### Technical Domain

The present disclosure relates to the field of nanomedicine, in particular for treating cancers. The present disclosure more specifically provides new methods of treating undesirable M2-polarized macrophages and/or inducing M1 macrophage polarization in a subject in need thereof, comprising administering to said subject a therapeutically effective amount of nanoparticles containing metallic elements.

### Background

Certain macrophages are important tumor-infiltrating cells and play pivotal roles in tumor growth and metastasis. In most solid tumors, the existence of macrophages is advantageous for tumor growth and metastasis. Recent studies indicate that tumor-associated macrophages (TAMs) show a M2 phenotype, so-called M2 polarized macrophages. These tumor-associated macrophages (TAM) produce interleukin IL-10 and transforming growth factor (TGF) β to suppress general antitumor immune responses. Meanwhile, TAMs promote tumor neo-angiogenesis by the secretion of pro-angiogenic factors and define the invasive microenvironment to facilitate tumor metastasis and dissemination. For these reasons, reducing the pool of M2-polarized macrophage has been considered as a relevant approach to anti-cancer therapy.

Several applications of nanomedicine (such as radioisotope-labeled or metallic nanoparticles) have been developed to improve the therapeutic index of radiation therapy by using nanomaterials as contrast agents to better deliver the radiation doses into tumor sites and/or as radiosensitizers, to enhance the dose deposition in tumors and reduce irradiation-related side-effects (Hainfeld et al., Br J Radiol 84(2011): 526-533; Dorsey et al., Transl Cancer Res 2(2013):280-291; Taupin et al., Phys Med Biol 60(2015):4449-4464; Carrière M, et al. Mutagenesis 32(2017):203-213; McQuaid et al., Sci Rep 6(2016):19442). Considering that the radiation dose absorbed by any tissue is proportional to relative atomic number (Z⁴-Z⁵) of the material, metallic nanoparticles containing high-Z atoms (such as gold or gadolinium) have been extensively investigated for their potential to improve radiotherapy. Under exposure to IR, heavy-metal based nanoparticles produce photons and Auger electrons that improve the total dose rate deposition into the tumors, induce the production of reactive oxygen species (ROS) and cause cellular damage in many tumors (e.g. melanoma, glioblastoma, breast and lung carcinomas) (Le Duc et al., ACS Nano 5(2011):9566-9574; Dufort et al., Small 11(2015):215-221; and Hainfeld et al., Phys Med Biol 49(2004):N309-N315).

In the present disclosure, the inventors now surprisingly identified a new effect of nanoparticles containing metallic elements previously used as radiosensitizing agents. They have indeed shown that certain nanoparticles containing metallic elements with a diameter below 10 nm, preferably below 6 nm, have a direct effect on macrophage cells, by triggering pro-inflammatory M1 macrophage polarization.

Moreover, and contrary to the indirect effect of radiosensitizing agents on cancer cells, which therapeutic effect is obtained only in combination with exposure of the patients to radiations, the inventors now identified that such nanoparticles may have a direct therapeutic effect that exerts on macrophage cells, without ionizing radiations. These findings trigger opportunities for developing novel anticancer therapeutic approaches, in particular when using such nanoparticles as an active ingredient in combination with other immunotherapies and/or for enhancing the effectiveness of conventional anticancer treatments (such as radiotherapy) through the reprogramming of tumor infiltrating macrophages.

Such nanoparticles could also be used to dictate macrophage fate and to treat human diseases triggered by excessive anti- or pro-inflammatory macrophage activation, such as inflammatory disorders, for example rheumatoid arthritis and atherosclerosis.

### Brief Description

The present disclosure relates to nanoparticles containing metallic elements, for use in a method of treating a condition associated with undesirable M2-polarized macrophages, in a subject in need thereof, the method comprising administering said nanoparticles containing metallic elements in said subject in need thereof; wherein said nanoparticles have a mean hydrodynamic diameter below 10 nm, for example between 1 and 8 nm, preferably between 2 and 6 nm.

In specific embodiments, said nanoparticles containing metallic elements are administered to said subject in an amount sufficient for in vivo inducing M1 macrophage polarization.

For example, said metallic elements are cationic elements, either as oxide and/or chalcogenide or halide or as a complex with a chelating agent, such as an organic chelating agent. More specifically, said metallic elements may advantageously be complexed with an organic chelating agent selected from chelating agents with carboxylic acid, amine, thiol, or phosphonate. Typically, said metallic elements are selected among the following elements: titanium, vanadium, chromium, manganese, iron, cobalt, nickel, copper, zinc, zirconium, niobium, molybdenum, hafnium, tantalum, tungsten, lanthanum, yttrium, and any other lanthanides.

In specific embodiments, said metallic elements are selected among those suitable for use as T1 contrast agent in magnetic resonance imaging (MRI), for example selected from the list consisting of: manganese, gadolinium, europium and terbium.

In other specific embodiments, said metallic elements are selected among those suitable for use as T2 contrast agent, typically selected from the following list: iron, manganese, cobalt, chromium and nickel.

In more specific embodiments, said metallic elements are further selected among high-Z elements, wherein said high-Z element is an element with an atomic Z number higher than 40, preferably higher than 50.

In other specific embodiments, said nanoparticles comprise chelates of metallic elements, for example chelates of rare earth elements. For example, said nanoparticles comprise
- polyorganosiloxane,
- chelates covalently bound to said polyorganosiloxane,
- metallic elements complexed by the chelates.

In more specific embodiments, said nanoparticles comprise
- polyorganosiloxanes with a silicium weight ratio of at least 8% of the total weight of the nanoparticle, preferably between 8% and 50%,
- chelates covalently bound to said polyorganosiloxane, in a proportion comprising between 5 and 100, preferably between 5 and 20 per nanoparticle, and,
- metallic elements complexed to the chelates.

For example, said nanoparticles may comprise chelates for complexing the metallic elements, obtained by grafting one or more of the following chelating agents on said nanoparticles: DOTA, DTPA, EDTA, EGTA, BAPTA, NOTA, DOTAGA, and DTPABA, or their mixtures.

In a particular embodiment of the above method, said subject is not exposed to ionizing radiations for radiotherapy within a period of at least 7 days, for example at least 10 days, following the administration of said nanoparticles containing metallic elements.

In a particular embodiment, said nanoparticles for use in the methods described herein are gadolinium-chelated polysiloxane nanoparticles of the following formula 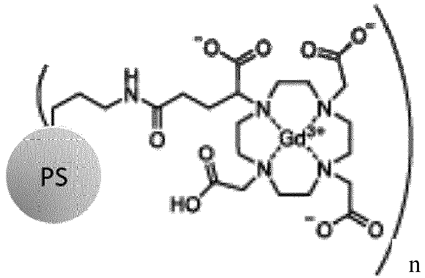 wherein PS is a matrix of polysiloxane and n is comprised between 5 and 50, and wherein the hydrodynamic diameter is comprised between 1 and 8 nm, typically between 2 and 6 nm.

In a preferred embodiment, said condition associated with undesirable M2-polarized macrophages is cancer, preferably cancer of head and neck, skin cancer, liver cancer, colon cancer, pancreatic cancer, breast cancer, ovary cancer, uterus cancer, cervical cancer, brain cancer, thyroid cancer, bladder cancer and renal cancer.

In specific embodiments, said method further comprises administering to said subject an immunotherapeutic treatment in combination with said metallic element containing nanoparticles, wherein the combined effect of (i) said metallic element containing nanoparticles in inducing M1 macrophage polarization and (ii) the immunotherapeutic treatment, produces a therapeutic effect on said cancer. More specifically, said immunotherapeutic treatment comprises administering a therapeutically efficient amount of one or more immunotherapeutic drugs selected from the group consisting of anti-PD1 or anti-PDL1 antibodies, (e.g. nivolumab, pembrolizumab, avelumab, durvalumab, cemiplimab, durvalumab or atezolizumab) or anti-CTLA-4 antibodies (e.g. ipilimumab).

Accordingly, in specific embodiments, said method further comprises exposing said subject to a radiotherapy, wherein the combined effect of (i) said metallic element containing nanoparticles in inducing M1 macrophage polarization and (ii) said radiotherapy, produces a therapeutic effect on said cancer.

In more specific embodiments, said radiotherapy further comprises administering a radiosensitizing amount of high-Z element containing nanoparticles, which may be the same or different as the metallic elements containing nanoparticles administered for inducing M1 macrophage polarization, and exposing said subject to an efficient dose of ionizing radiations for radiotherapy, wherein the combined effect of (i) said high-Z element containing nanoparticles in inducing M1 macrophage polarization, (ii) said high-Z element containing nanoparticles as radiosensitizing agent, and (iii) the ionizing radiations, produces a therapeutic effect on said cancer. Typically, a radiosensitizing amount of high-Z element containing nanoparticles is comprised between 15mg/kg and 200mg/kg per injection.

In other embodiments, a condition associated with undesirable M2-polarized macrophages is an inflammatory disorder, including progressive fibrotic diseaases, such as for example idiopathic pulmonary fibrosis, hepatic fibrosis systemic sclerosis, allergy, asthma and atherosclerosis.

In specific embodiments, said nanoparticles are administered to the patient by intravenous injection of a therapeutically efficient amount of a suspension of said nanoparticles.

### Legends to Figures

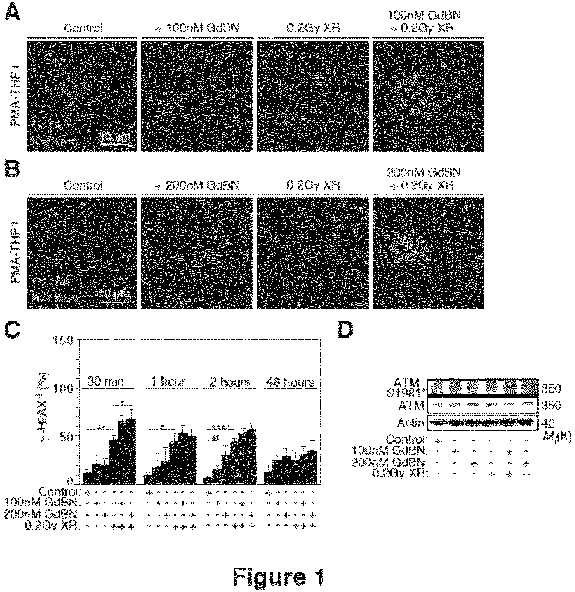

**Figure 1****. GdBN alone or in combination with ionizing radiation induce DNA damage and DNA-damage response in anti-inflammatory macrophages.**
   (A, B) Fluorescent microscopy images of γH2AX⁺ foci detected on PMA-treated THP1 human macrophages after 30 minutes (A) or 2 hours (B) treatment with control, 100nM GdBN, 0.2 Gy XR or 100nM GdBN+ 0.2 Gy XR. Images are representative of 3 independent experiments.
   (C) Percentage of γH2AX foci positive cells detected 30 minutes, 1 hour, 2 hour or 48 hours after treatment. Data are means ± S.E.M from three independent experiments. ANOVA test (****P< 0.0001, **P<0.01, *P<0.05).
   (D) Expression of ATMS1981* and ATM detected 30 minutes after treatment was analyzed by western blot. Representative western blot of 3 independent experiments is shown.

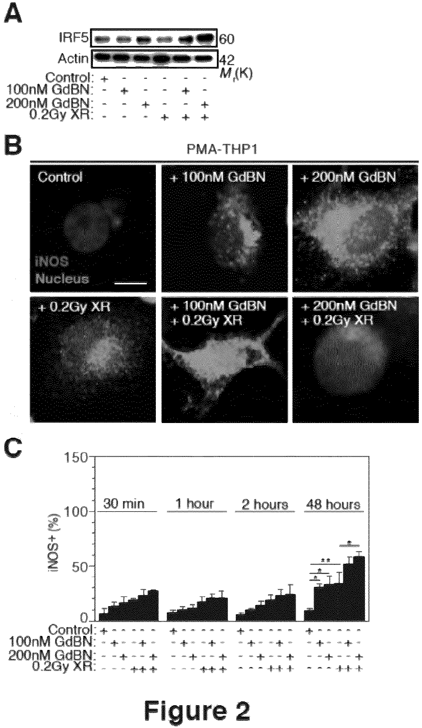

**Figure 2****. GdBN alone or in combination with ionizing radiation trigger pro-inflammatory macrophage reprogramming.**
   (A) Expression of IRF5 detected after 48 hours treatment of PMA-treated human THP1 monocytes with control, 100 nM GdBN, 0.2 Gy XR or 100nM GdBN + 0.2 Gy XR was analyzed by western blot. Representative western blot of 3 independent experiments is shown.
   (B) Fluorescent microscopy images of iNOS expression on PMA-treated THP1 human macrophages after 48 hours treatment with control, 100 nM GdBN, 200 nM GdBN, 0.2 Gy XR,100 nM GdBN + 0.2 Gy XR or 200 nM GdBN + 0.2 Gy XR. Images are representative of 3 independent experiments.
   (C) Percentage of iNOS positive cells detected 30 minutes, 1 hour, 2 hours and 48 hours after irradiation. Data are means ± S.E.M from three independent experiments. ANOVA test (****P< 0.0001, **P<0.01, *P<0.05).

### Detailed Description

The present disclosure relates to nanoparticles containing metallic elements, for use in a method of treating a condition associated with undesirable M2-polarized macrophages in a subject in need thereof, the method comprising administering said nanoparticles containing metallic elements in said subject in need thereof,
wherein said nanoparticles have a mean hydrodynamic diameter below 10 nm, typically between 1 and 8 nm, preferably below 6 nm, for example between 2 and 6 nm.

As used herein, the term "treating" or "treatment" refers to one or more of (1) inhibiting the disease; for example, inhibiting a disease, condition or disorder in an individual who is experiencing or displaying the pathology or symptomatology of the disease, condition or disorder (i.e., arresting further development of the pathology and/or symptomatology); and (2) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease or reducing or alleviating one or more symptoms of the disease. In particular, with reference to the treatment of a tumor, the term "treatment" may refer to the inhibition of the growth of the tumor, or the reduction of the size of the tumor.

### The nanoparticles for use in the methods according to the disclosure

The present disclosure follows from the surprising advantages, demonstrated by the inventors, of a direct effect of certain nanoparticles containing metallic elements to induce M1 macrophage polarization.

In specific embodiments, the method thus comprises administering the nanoparticles in an amount sufficient for in vivo inducing M1 macrophage polarization.

The advantageous effects of the method of the present disclosure are linked in particular to two features of the nanoparticles:
(i) they contain metallic elements, typically complexes of metallic cations;
(ii) they have a small mean hydrodynamic diameter.

Nanoparticles with a mean hydrodynamic diameter for example of between 1 and 10 nm, and even more preferably between 1 and 8 nm or for example between 2 and 6 nm, typically, around 3 nm further allowing excellent passive targeting in tumors after intravenous injection, and a rapid renal elimination (and therefore low toxicity) will be advantageously selected.

The size distribution of the nanoparticles is, for example, measured using a commercial particle sizer, such as a Malvern Zêtasizer Nano-S particle sizer based on PCS (Photon Correlation Spectroscopy). This distribution is characterized by a mean hydrodynamic diameter between 1 and 10 nm and typically between 2 and 6 nm.

For the purposes of the present disclosure, the term "mean hydrodynamic diameter" or "mean diameter" is intended to mean the harmonic mean of the diameters of the particles. A method for measuring this parameter is also described in standard ISO 13321:1996.

In specific embodiments said metallic elements for use in the methods of the present disclosure are selected among the following list: titanium, vanadium, chromium, manganese, iron, cobalt, nickel, copper, zinc, zirconium, niobium, molybdenum, hafnium, tantalum, tungsten, lanthanum, yttrium, and any other lanthanides.

In a more specific embodiment, said metallic elements are selected among those suitable for use in magnetic resonance imaging (MRI) as T1 contrast agent, for example from the list consisting of: manganese, gadolinium, europium and terbium.

In another specific embodiment, said metallic elements are selected among those suitable for use as T2 contrast agent, typically selected from the following list: iron, manganese, cobalt, chromium and nickel.

In specific embodiments, said metallic elements further have radiosensitizing properties for use in radiotherapy. As used herein, the term "radiosensitizing" would be readily understood by one of ordinary skill in the art and generally refers to the process of increasing the sensitivity of the cancer cells to radiation therapy (e.g., photon radiation, electron radiation, proton radiation, heavy ion radiation, and the like).

In more specific embodiments, said metallic elements are selected from high-Z element. Said high-Z element as used herein is an element with an atomic Z number higher than 40, for example higher than 50.

In specific embodiments, said high-Z element is selected among the heavy metals, and more preferably, Au, Ag, Pt, Pd, Sn, Ta, Zr, Tb, Tm, Ce, Dy, Er, Eu, La, Nd, Pr, Lu, Yb, Bi, Hf, Ho, Pm, Sm, In, and Gd, and mixtures thereof.

The metallic elements are preferably cationic elements, either comprised in the nanoparticles as oxide and/or chalcogenide or halide or as complexes with chelating agents, such as organic chelating agents.

In another embodiment, which may be combined with previous embodiments, the nanoparticles can be also advantageously used as an imaging or a contrast agent, for example, in image-guided radiation therapy.

As used herein, the term "contrast agent" is intended to mean any product or composition used in medical imaging for the purpose of artificially increasing the contrast making it possible to visualize a particular anatomical structure (for example certain tissues or organs) or pathological anatomical structures (for example tumors) with respect to neighboring or non-pathological structures. The term "imaging agent" is intended to mean any product or composition used in medical imaging for the purpose of creating a signal making it possible to visualize a particular anatomical structure (for example certain tissues or organs) or pathological anatomical structures (for example tumors) with respect to neighboring or non-pathological structures. The principle of how the contrast or imaging agent operates depends on the imaging technique used.

In some embodiments, the imaging is performed using magnetic resonance imaging (MRI), computed tomography imaging, positron emission tomography imaging, or any combination thereof.

Advantageously, it will be possible to combine the use of the nanoparticles in the method of treatment of the disclosure, and for an in vivo detection by MRI, enabling, for example, monitoring of the therapeutic treatment as described in the present disclosure.

Preferably, only lanthanides, including at least 50% by weight of gadolinium (Gd), of dysprosium (Dy), of lutetium (Lu), for bismuth (Bi) or of holmium (Ho), or mixtures thereof, for example at least 50% by weight of gadolinium, will be chosen as metallic elements in the nanoparticles.

According to one variant, use will be made of nanoparticles in which the part containing lanthanides contains, at its periphery, lanthanides which cause an MRI signal, for example gadolinium, and at least one high-Z element (e.g. Bi) in its central part. Radiation-absorbing high-Z metals with a very high atomic number may therefore be located at the center of the core of the nanoparticle.

In one particular embodiment, the nanoparticles that can be used according to the disclosure are characterized in that they comprise at least one contrast agent for T1 MRI imaging, and at least one other imaging or contrast agent suitable for one of the following imaging techniques:
- PET or SPECT scintigraphy,
- fluorescence in the visible range or in the near-infrared range,
- X-ray tomodensitometry.

In specific embodiments, said metallic elements are cationic elements complexed with organic chelating agents, for example selected from chelating agents with carboxylic acid, amine, thiol, or phosphonate groups.

In preferred embodiment, the nanoparticles further comprise a biocompatible coating in addition to the metallic element, and, optionally, the chelating agents. Agent suitable for such biocompatible includes without limitation biocompatible polymers, such as polyethylene glycol, polyethyleneoxide, polyacrylamide, biopolymers, polysaccharides, or polysiloxane.

In particular embodiments, the nanoparticles are chosen such that they have a relaxivity r1 per particle of between 50 and 5000 mM⁻¹.s⁻¹ (at 37°C and 1.4 T) and/or a Gd weight ratio of at least 5 %, for example between 5 % and 30 %.

In one specific embodiment, said nanoparticles with a very small hydrodynamic diameter, for example between 1 and 10 nm, preferably between 2 and 6 nm, are nanoparticles comprising chelates of metallic elements, for example chelates of rare earth elements, and more preferably chelates of gadolinium or bismuth.

In specific embodiments that may be preferably combined with any of the previous embodiments, said nanoparticles comprises
- polyorganosiloxane,
- chelating agents covalently bound to said polyorganosiloxane,
- metallic elements complexed by the chelating agent.

As used herein, the term "chelating agent" refers to a group capable of complexing one or more metal ions.

Exemplary chelating agents include, but not limited to, 1,4,7-triazacyclononanetriacetic acid (NOTA), I,4,7,10-tetraazacyclododecane-I,4,7,10-tetraacetic acid (DOTA), 1,4,7-triazacyclononane-I-glutaric acid-4,7-diacetic acid (NODAGA), ethylene diamine tetra-acetic acid (EDTA), diethylene triaminepentaacetic acid (DTPA), cyclohexyl-I,2-diaminetetraacetic acid (CDTA), ethyleneglycol-0,0'-bis(2-aminoethyl)-N,N,N',N'-tetraacetic acid (EGTA), N,N-bis(hydroxybenzyl)- ethylenediamine-N,N'-diacetic acid (HBED), triethylene tetramine hexaacetic acid (TTHA), hydroxyethyidiamine tnacetic acid (HEDTA), 1,4,8,11- tetraazacyclotetradecane-N,N',N",N"'-tetraacetic acid (TETA), and 1, 4,7,10-tetraaza- I,4,7,10-tetra-(2-carbamoyl methyl)-cyclododecane (TCMC) and 1,4,7,10-tetraazacyclododececane,1-(glutaric acid)-4,7,10-triacetic acid (DOTAGA).

In some embodiments, said chelating agent is selected among the following: 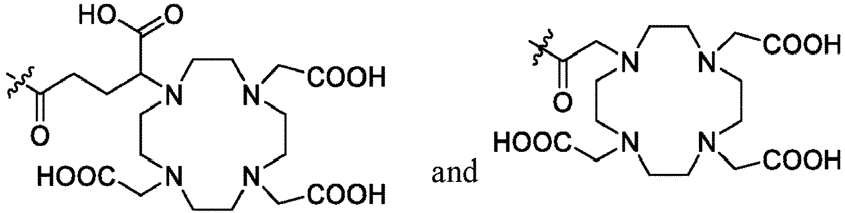 wherein the wavy bond indicates the bond connecting the chelating agent to a linking group of a biocompatible coating forming the nanoparticle.

In a specific embodiment, that may be preferably combined with the previous embodiment, said chelates of rare earth element are chelates of gadolinium and/or bismuth, preferably DOTA or DOTAGA chelating Gd³⁺ and/or Bi.

In specific and preferred embodiments, the ratio of metallic element per nanoparticle, for example the ratio of rare earth elements, e.g. gadolinium (optionally as chelated with DOTAGA) per nanoparticle, is between 3 and 100, preferably between 5 and 50, typically around 15.

For imaging by scintigraphy, the nanoparticles may additionally comprise a radioactive isotope that can be used in scintigraphy, and that is preferably chosen from the group consisting of the radioactive isotopes of In, Tc, Ga, Cu, Zr, Y or Lu, for example: ¹¹¹In, ^{99m}Tc, ⁶⁷Ga, ⁶⁸Ga, ⁶⁴Cu, ⁸⁹Zr, ⁹⁰Y or ¹⁷⁷Lu.

For fluorescence in the near-infrared range, the nanoparticles may additionally comprise a lanthanide chosen from Nd, Yb or Er.

For fluorescence in the visible range, the nanoparticles may additionally comprise a lanthanide chosen from Eu or Tb can be used.

For fluorescence in the near-infrared range, the nanoparticles may additionally comprise an organic fluorophore chosen from Cyanine 5.5, Cyanine 7, Alexa 680, Alexa 700, Alexa 750, Alexa 790, Bodipy.

In specific embodiments, the hybrid nanoparticles are of core-shell type. Nanoparticles of core-shell type, based on a core consisting of a rare earth oxide and of an optionally functionalized polyorganosiloxane matrix are known (see in particular WO 2005/088314, WO 2009/053644).

The nanoparticles may further be functionalized with molecules which allow targeting of the nanoparticles to specific tissues. Said agents can be coupled to the nanoparticle by covalent couplings, or trapped by non-covalent bonding, for example by encapsulation or hydrophilic/hydrophobic interaction or using a chelating agent.

In one specific embodiment, use is made of hybrid nanoparticles comprising:
- a polyorganosiloxane (POS) matrix including, rare earth cations Mⁿ⁺, n being an integer between 2 and 4, optionally partly in the form of a metal oxide and/or oxyhydroxide, optionally associated with doping cations D^{m+}, m being an integer between 2 and 6, D preferably being a rare earth metal other than M, an actinide and/or a transition element;
- a chelate covently bound to the POS via a covalent bond -Si-C-,
- the Mⁿ⁺ cations and, where appropriate, D^{m+} cations being complexed by the chelates.

In the case of a structure of core-shell type, the POS matrix forms the superficial layer surrounding the metal cation-based core. Its thickness can range from 0.5 to 10 nm, and can represent from 25% to 75% of the total volume.

The POS matrix acts as protection for the core with respect to the external medium (in particular protection against hydrolysis) and it optimizes the properties of the contrast agents (luminescence, for example). It also allows the functionalization of the nanoparticle, via the grafting of chelating agents and of targeting molecules.

### Ultrafine nanoparticles

In a specific embodiment, said nanoparticles are gadolinium-chelated polysiloxane nanoparticles of the following formula 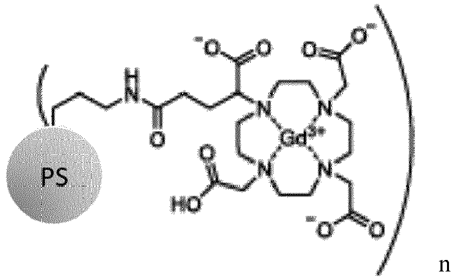 wherein PS is a matrix of polysiloxane, and wherein n is comprised between 5 and 50, for example between 5 and 20, and wherein the hydrodynamic diameter is comprised between 2 and 6 nm (hereafter referred as "ultrafine nanoparticles").

In a more specific embodiment, said gadolinium-chelated polysiloxane nanoparticle is nanoparticle of the above formula having a hydrodynamic diameter of 4± 2 nm and a mass between 5 and 30 kDa.

In one more particularly preferred embodiment, owing in particular to their very small size and rigid structure, such ultrafine nanoparticles that can be used according to the disclosure are obtained by a top-down synthesis route comprising the steps of:
a. obtaining a metal (M) oxide core, wherein M is a metallic element as described previously,
b. adding a polysiloxane shell around the M oxide core, for example via a sol gel process,
c. grafting a chelating agent to the POS shell, so that the chelating agent is bound to said POS shell by an -Si-C- covalent bond, thereby obtaining a core-shell precursor nanoparticle, and,
d. purifying and transferring the core-shell precursor nanoparticle in an aqueous solution,
wherein the grafted agent is in sufficient amount to dissolve the metal (M) oxide core at step d. and to complex the cationic form of (M) thereby reducing the mean hydrodynamic diameter of the resulting hybrid nanoparticle to a mean diameter less than 10 nm, for example, between 1 and 8 nm, typically less than 6 nm, for example between 2 and 6 nm.

These nanoparticles obtained according to the mode described above do not comprise a core of metal oxide encapsulated by at least one coating. More details regarding the synthesis of these nanoparticles are given in the next Section.

This top-down synthesis method results in observed sizes typically of between 1 and 8 nm, more specifically between 2 and 6nm.

Alternatively, another "one-pot" synthesis method is described in the next section to prepare said ultrafine or "core-free" nanoparticles with a mean diameter less than 10nm, for example, between 1 and 8 nm, typically between 2 and 6 nm.

Preferably, the ultrafine or "core-free" nanoparticles for use in the method according to the disclosure have a relaxivity r1 per Mⁿ⁺ ion greater than 5 mM⁻¹.s⁻¹ (at 37°C) (of Mⁿ⁺ ion), preferentially 10 mM⁻¹.s⁻¹ (at 37°C) (of Mⁿ⁺ ion), for a frequency of 20 MHz. For example, they have a relaxivity r1 per nanoparticle of between 50 and 5000 mM⁻¹·s⁻¹. Even better still, these nanoparticles have a relaxivity r1 per Mⁿ⁺ ion at 60 MHz which is greater than or equal to the relaxivity r1 per Mⁿ⁺ ion at 20 MHz. The relaxivity r1 considered here is a relaxivity per Mⁿ⁺ (for example gadolinium) ion. r1 is extracted from the following formula: 1/T1 = [1/T1]water + r1[Mⁿ⁺].

Further details regarding these ultrafine or core-free nanoparticles, the processes for synthesizing them and their uses are described in patent application WO 2011/135101, WO2018/224684 or WO2019/008040, which is incorporated by way of reference.

### Process for obtaining preferred embodiments of nanoparticles for use according to the disclosure

Generally, those skilled in the art will be able to easily produce nanoparticles used according to the invention. More specifically, the following elements will be noted:
For nanoparticles of core-shell type, based on a core of lanthanide oxide or oxyhydroxide, use will be made of a production process using an alcohol as solvent, as described for example in P. Perriat et al., J. Coll. Int. Sci, 2004, 273, 191; O. Tillement et al., J. Am. Chem. Soc., 2007, 129, 5076 and P. Perriat et al., J. Phys. Chem. C, 2009, 113, 4038.

For the POS matrix, several techniques can be used, derived from those initiated by Stoeber (Stoeber, W; J. Colloid Interf Sci 1968, 26, 62). Use may also be made of the process used for coating as described in Louis et al. (Louis et al., 2005, Chemistry of Materials, 17, 1673-1682) or international application WO 2005/088314.

In practice, synthesis of ultrafine nanoparticles is for example described in Mignot et al Chem. Eur. J. 2013, 19, 6122-6136: Typically, a precursor nanoparticle of core/shell type is formed with a lanthanide oxide core (via the modified polyol route) and a polysiloxane shell (via sol/gel); this object has, for example, a hydrodynamic diameter of around 5-10 nm. A lanthanide oxide core of very small size (adjustable less than 10 nm) can thus be produced in an alcohol by means of one of the processes described in the following publications: P. Perriat et al., J. Coll. Int. Sci, 2004, 273, 191; O. Tillement et al., J. Am. Chem. Soc., 2007, 129, 5076 and P. Perriat et al., J. Phys. Chem. C, 2009, 113, 4038.

These cores can be coated with a layer of polysiloxane according to, for example, a protocol described in the following publications: C. Louis et al., Chem. Mat., 2005, 17, 1673 and O. Tillement et al., J. Am. Chem. Soc., 2007, 129, 5076.

Chelating agents specific for the intended metal cations (for example DOTAGA for Gd³⁺) are grafted to the surface of the polysiloxane; it is also possible to insert a part thereof inside the layer, but the control of the formation of the polysiloxane is complex and simple external grafting gives, at these very small sizes, a sufficient proportion of grafting.

The nanoparticles are separated from the synthesis residues by means of a method of dialysis or of tangential filtration, on a membrane comprising pores of appropriate size.

The core is destroyed by dissolution (for example by modifying the pH or by introducing complexing molecules into the solution). This destruction of the core then allows a scattering of the polysiloxane layer (according to a mechanism of slow corrosion or collapse), which makes it possible to finally obtain a polysiloxane object with a complex morphology, the characteristic dimensions of which are of the order of magnitude of the thickness of the polysiloxane layer, i.e. much smaller than the objects produced up until now.

Removing the core thus makes it possible to decrease from a particle size of approximately 5-10 nanometers in diameter to a size below 8nm, for example about 3 nanometers. Furthermore, this operation makes it possible to increase the number of M (e.g. gadolinium) per nm3 in comparison with a theoretical polysiloxane nanoparticle of the same size but comprising M (e.g. gadolinium) only at the surface. The number of M for a nanoparticle size can be evaluated by virtue of the M/Si atomic ratio measured by EDX. Typically, this number of M per ultrafine nanoparticle may be comprised between 5 and 50.

Targeting molecules can be grafted onto these nanoparticles for example using coupling by peptide bonding on an organic constituent of the nanoparticle, as described in Montalbetti, C.A.G.N, Falque B. Tetrahedron 2005, 61, 10827-10852.

Use may also be made of a coupling method using "click chemistry", Jewett, J.C.; Bertozzi, C.R. Chem. Soc. Rev. 2010, 39, 1272-1279, and involving groups of the type:
-N3, -CN or -C≡CH, or one of the following groups:

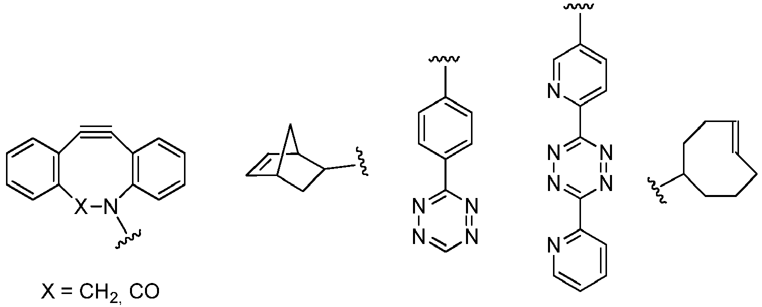

In one specific embodiment, the nanoparticle for use according to the disclosure comprises a chelating agent which has an acid function, for example DOTA or DOTAGA. The acid function of the nanoparticle is activated for example using EDC/NHS (1-ethyl-3-(3-dimethylaminopropyl)carbodiimide / N-hydrosuccinimide) in the presence of an appropriate amount of targeting molecules. The nanoparticles thus grafted are then purified, for example by tangential filtration.

According to a specific embodiment, the nanoparticles for use according to the present disclosure are obtained by a synthesis method ("one-pot synthesis method") comprising the mixing of at least one hydroxysilane or alkoxysilane which is negatively charged at physiological pH and at least one chelating agent chosen from polyamino polycarboxylic acids with
- at least one hydroxysilane or alkoxysilane which is neutral at physiological pH, and/or
- at least one hydroxysilane or alkoxysilane which is positively charged at physiological pH and comprises an amino function,
wherein:
- the molar ratio **A** of neutral silane(s) to negatively charged silane(s) is defined as follows: 0 ≤ **A** ≤ 6, preferably 0.5 ≤ **A** ≤ 2;
- the molar ratio **B** of positively charged silane(s) to negatively charged silane(s) is defined as follows: 0 ≤ **B** ≤ 5, preferably 0.25 ≤ **B** ≤ 3;
- the molar ratio **C** of neutral and positively charged silanes to negatively charged silane(s) is defined as follows 0 < **C** ≤ 8, preferably 1 ≤ **C** ≤ 4.

According to a more specific embodiment of such one pot synthesis method, the method comprises the mixing of at least one alkoxysilane which is negatively charged at physiological pH, said alkoxysilane being chosen among APTES-DOTAGA, TANED, CEST and mixtures thereof, with
- at least alkoxysilane which is neutral at physiological pH, said alkoxysilane being chosen among TMOS, TEOS and mixtures thereof, and/or
- APTES which is positively charged at physiological pH,
wherein:
- the molar ratio **A** of neutral silane(s) to negatively charged silane(s) is defined as follows: 0 ≤ **A** ≤ 6, preferably 0.5 ≤ **A** ≤ 2;
- the molar ratio **B** of positively charged silane(s) to negatively charged silane(s) is defined as follows: 0 ≤ **B** ≤ 5, preferably 0.25 ≤ **B** ≤ 3;
- the molar ratio **C** of neutral and positively charged silanes to negatively charged silane(s) is defined as follows 0 < **C** ≤ 8, preferably 1 ≤ **C** ≤ 4.

According to a specific embodiment, the one pot synthesis method comprises the mixing of APTES-DOTAGA which is negatively charged at physiological pH with
- at least one alkoxysilane which is neutral at physiological pH, said alkoxysilane being chosen among TMOS, TEOS and mixtures thereof, and/or
- APTES which is positively charged at physiological pH,
wherein:
- the molar ratio **A** of neutral silane(s) to negatively charged silane(s) is defined as follows: 0 ≤ **A** ≤ 6, preferably 0.5 ≤ **A** ≤ 2;
- the molar ratio **B** of positively charged silane(s) to negatively charged silane(s) is defined as follows: 0 ≤ **B** ≤ 5, preferably 0.25 ≤ **B** ≤ 3;
- the molar ratio C of neutral and positively charged silanes to negatively charged silane(s) is defined as follows 0 < **C** ≤ 8, preferably 1 ≤ **C** ≤ 4.

### AguIX Nanoparticles

In a more specific embodiment, said gadolinium-chelated polysiloxane nanoparticle for use in the method of the present disclosure is the core-free ultrafine AgulX nanoparticle of the formula below 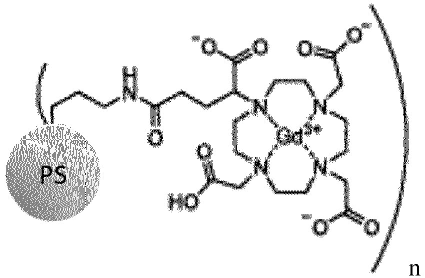 wherein PS is polysiloxane and wherein n is, on average, about 10, and having a hydrodynamic diameter of 4± 2 nm and a mass of about 10± 1 kDa.

Said AgulX nanoparticle can also be described by the average chemical formula:

(GdSi₄₋₇C₂₄₋₃₀N₅₋₈O₁₅₋₂₅H₄₀₋₆₀, 5-10H₂O)ₓ

Methods for synthesis of AguIX nanoparticles are also described in the examples below.

### Pharmaceutical formulations of the nanoparticles for use according to the disclosed methods

When employed as pharmaceuticals, the compositions comprising said nanoparticles for use as provided herein can be administered in the form of pharmaceutical formulation of a suspension of nanoparticles. These formulations can be prepared as described herein or elsewhere, and can be administered by a variety of routes, depending upon whether local or systemic treatment is desired and upon the area to be treated.

In particular, said pharmaceutical formulations for use as described herein, contain, as the active ingredient, a suspension of nanoparticles, as provided herein, in combination with one or more pharmaceutically acceptable carriers (excipients). In making a pharmaceutical formulation provided herein, the nanoparticle composition may be, for example, mixed with an excipient or diluted by an excipient. When the excipient serves as a diluent, it can be a solid, semi-solid, or liquid material, which acts as a vehicle, carrier, or medium for the nanoparticle composition.

Thus, the pharmaceutical formulations can be in the form of powders, lozenges, elixirs, suspensions, emulsions, solutions, syrups, aerosols (as a solid or in a liquid medium), sterile injectable solutions, sterile packaged powders, and the like.

In specific embodiments, said pharmaceutical formulation for use as described herein, is sterile lyophilized powder, contained in a pre-filled vial to be reconstituted, for example in an aqueous solution for intravenous injection. In specific embodiments, said lyophilized powder comprises, as the active ingredient, an efficient amount of said nanoparticles containing metallic elements, typically gadolinium-chelated polysiloxane based nanoparticles, and more specifically AguIX nanoparticles as described herein. In certain specific embodiments, said lyophilized powder contains about between 280 and 320 mg of AguIX per vial, for example 300 mg of AguIX per vial. Such powder may further contain one or more additional excipients, and in particular CaCl₂, for example between 0.5 and 0.80 mg of CaCl₂, typically 0.66mg of CaCl₂.

Said lyophilized powder may be reconstituted in an aqueous solution, typically water for injection. Accordingly, in specific embodiments, said pharmaceutical solution for use according to the present disclosure is a solution for injection, comprising, as the active ingredient, an efficient amount of said nanoparticles containing metallic elements, typically gadolinium-chelated polysiloxane based nanoparticles, and more specifically AguIX nanoparticles as described herein.

For example, said solution for injection for use in the methods as disclosed herein is a solution of gadolinium-chelated polysiloxane based nanoparticles, typically AguIX nanoparticles, at a concentration between 50 and 150 mg/mL, for example 80 and 120 mg/mL, typically 100mg/mL, optionally comprising one or more additional pharmaceutically acceptable excipient, for example between 0.1 and 0.3mg/mL of CaCl₂, typically 0.22 mg/mL of CaCl₂.

### Methods of use of the present disclosure

The methods and use according to the present disclosure are intended to treat conditions associated with undesirable M2-polarized macrophages in a subject, typically a human subject.

The term "patient" and "subject" which are used herein interchangeably refer to any member of the animal kingdom, including mammals and invertebrates. For example, mice, rats, other rodents, rabbits, dogs, cats, swine, cattle, sheep, horses, primates, fish, and humans. Preferably, the subject is a mammal, or a human being, including for example a subject that has a condition associated with undesirable M2-polarized macrophages.

The term "M2-polarized macrophages" also called "alternatively activated M2 macrophages" refers to macrophages which are activated by Th2 cytokines (e.g. interleukin IL-4, IL-10, and IL-13). The alternatively activated (M2) macrophages appear to be involved in immunosuppression and tissue repair.

Accordingly, as used herein, a condition associated with undesirable M2 polarized macrophage refer to any condition which is induced, enhanced, or worsened in the presence of M2-polarized macrophages, and/or can be treated by reducing M2 polarized macrophage and/or inducing M1 macrophage polarization.

Other conditions associated with undesirable M2-polarized macrophages include without limitation inflammatory disorders, including progressive fibrotic diseases, such as for example idiopathic pulmonary fibrosis, hepatic fibrosis, systemic sclerosis, allergy, asthma and atherosclerosis. Indeed, it has been shown that certain inflammatory disorders may advantageously be treated with agents capable of triggering M1 pro-inflammatory macrophages and/or decreasing M2-polarized macrophages (L. Meziani et al., Eur Respir J. 51(2018):pii1702120; L. Meziani et al., Oncoimmunology 7(2018):e1494488, F. Tacke J Hepatol 66(2017):1300-1312, PJ Wermuth and S.A Jimenez Clin Transl Med 2015 4(2015):2. PF Ma J Hepathol 67(2017):770-779, D Zhou et al., Immunol Cell Biol 2017 95(10):864-869).

The inventors have identified that certain nanoparticles containing metallic elements, as disclosed in the previous Sections, favor pro-inflammatory macrophage reprogramming.

Hence, in specific embodiments, the present disclosure relates to a method of decreasing the amount of M2-polarized macrophages, typically tumor-associated macrophages in tumors, and/or increasing the amount of M1-polarized macrophages, in a subject in need thereof, said method comprising administering to the subject a therapeutically effective amount of said nanoparticles containing metallic elements, wherein said nanoparticles have a mean hydrodynamic diameter below 10 nm, preferably below 6 nm, for example between 2 and 6 nm.

The present disclosure further relates to a method for treating a condition associated with undesirable M2-polarized macrophages in a subject in need thereof, the method comprising administering to the subject a therapeutically efficient amount of said nanoparticles containing metallic elements, wherein said nanoparticles have a mean hydrodynamic diameter below 10 nm, preferably below 6 nm, for example between 2 and 6 nm.

As used herein the expression "reducing M2-polarized macrophages" means that the nanoparticles containing metallic elements causes a decrease in the M2 macrophage activation pool and/or increase in MI macrophages pool, preferably a decrease in the M2 macrophage activation pool and an increase in MI macrophages pool. Thus, the M1/M2 ratio increases. This can be indicated for example, by changes in the levels of factors that are associated with MI and M2 macrophages.

The disclosure thus also relates to a method of increasing M1 macrophages pool in a subject suffering from conditions associated with undesirable M2-polarized macrophages comprising administering to the subject a therapeutically effective amount of said nanoparticles containing elements as disclosed in the previous sections.

Nanoparticles containing metallic elements were previously disclosed as radiosensitizing agents, for their therapeutic use solely in combination with ionizing radiations. For a use of nanoparticles as radiosensitizing agent, it is generally considered that said nanoparticles should be administered shortly before exposure to ionizing radiations (e.g. less than 24 hours). The inventors now show that the effect of said nanoparticles in inducing M1 macrophage polarization can be exerted without ionizing radiations.

Thus, in specific embodiments of the use of the nanoparticles, said subject is not exposed to ionizing radiations for radiotherapy within a period of at least 7 days, for example at least 10 days, following the first administration of said nanoparticles containing metallic elements to said patient.

### Methods for treating cancer

In specific embodiments of the use of the nanoparticles according to the present disclosure, said condition associated with undesirable M2-polarized macrophage is cancer.

In some embodiments, the cancer is a solid tumor. In some embodiments, the cancer is selected from the group consisting of lung cancer, brain cancer, cancer of the head and neck, cervical cancer, pancreatic cancer, breast cancer, prostate cancer, liver cancer, colon cancer, endometrial cancer, bladder cancer, skin cancer (e.g., melanoma), renal cancer, and gastric cancer. In some embodiments, the cancer is a metastatic cancer.

More preferably, in some embodiments, the cancer is selected from cancer of head and neck, skin cancer, liver cancer, colon cancer, pancreatic cancer, breast cancer, ovary cancer, uterine cancer, cervical cancer, brain cancer, thyroid cancer, bladder cancer and renal cancer.

In certain embodiments, the presence or absence of M2-polarized macrophage in tumors in a subject may be detected, for example based on CD68 expression, as described in M. Heusinkveld and SH Van der Burg. J Transl Med 2011 9(2011):216, Q. Wu et al., Cell Death Diff 24(2017):1632-1644. The nanoparticles for use as disclosed herein may be administered as the sole active ingredient or in conjunction with, e.g. as an adjuvant to or in combination to, other drugs e.g anti-inflammatory agents or cytotoxic, anti-proliferative, chemotherapy or anti-tumor agents, e.g. for the treatment or prevention of cancer disorders, as mentioned above.

Suitable cytotoxic, anti-proliferative or anti-tumor agents may include without limitation cisplatin, doxorubicin, taxol, etoposide, irinotecan, topotecan, paclitaxel, docetaxel, epothilones, tamoxifen, 5-fluorouracil, methotrexate, temozolomide, cyclophosphamide, tipifarnib, gefitinib, erlotinib, imatinib, gemcitabine, uracil mustard, chlormethine, ifosfamide, melphalan, chlorambucil, pipobroman, triethylenemelamine, busulfan, carmustine, lomustine, streptozocin, dacarbazine, floxuridine, cytarabine, 6-mercaptopurine, 6- thioguanine, fludarabine phosphate, oxaliplatin, folinic acid, pentostatin, vinblastine, vincristine, vindesine, bleomycin, dactinomycin, daunorubicin, epirubicin, idarubicin, mithramycin, deoxycoformycin, mitomycin-C, L-asparaginase, teniposide.

In other embodiments, the additional therapeutic agent is an immunotherapeutic agent. Exemplary immunotherapeutic agents include, but are not limited to, anti-PD1 or anti-PDL1 antibodies, (e.g. nivolumab, pembrolizumab, avelumab, durvalumab, cemiplimab, durvalumab or atezolizumab), anti-CD52 antibodies (alemtuzumab), anti-CTLA-4 antibodies (e.g. ipilimumab), anti-CD20 antibodies (ofatumumab, rituximab), human type I interferon-a (i.e. IFN-a), and interleukin-2.

In some embodiments, the additional therapeutic agent is administered simultaneously with a pharmaceutical formulation of nanoparticles as provided herein. In some embodiments, the additional therapeutic agent is administered after administration of the pharmaceutical formulation of nanoparticles as provided herein. In some embodiments, the additional therapeutic agent is administered prior to administration of the pharmaceutical formulation of nanoparticles. In some embodiments, the pharmaceutical formulation of nanoparticles provided herein is administered during a surgical procedure. In some embodiments, the a pharmaceutical formulation of nanoparticles provided herein is administered in combination with an additional therapeutic agent during a surgical procedure.

The additional therapeutic agents provided herein can be effective over a wide dosage range and are generally administered in an effective amount. It will be understood, however, that the amount of the therapeutic agent actually administered will usually be determined by a physician, according to the relevant circumstances, including the condition to be treated, the chosen route of administration, the actual compound administered, the age, weight, and response of the individual subject, the severity of the subject's symptoms, and the like.

For example, a method of the disclosure further comprises administering to said subject an immunotherapeutic treatment in combination with said nanoparticles containing metallic elements, wherein the combined effect of (i) said nanoparticles containing metallic elements in inducing M1 macrophage polarization and (ii) the immunotherapeutic treatment, produces a therapeutic effect on said cancer.

In a specific embodiment, the nanoparticles for use as disclosed may be used in combination with one or more immunotherapeutic agents, preferably selected among those capable of enhancing the effect of inducing M1 macrophage polarization. Examples of such immunotherapeutic agents include without limitation anti-PD1 or anti-PDL1 antibodies, (e.g. nivolumab, pembrolizumab, avelumab, durvalumab, cemiplimab, durvalumab or atezolizumab) or anti-CTLA-4 antibodies (e.g. ipilimumab).

In addition, the inventors further noticed a synergistic effect of the novel effect of said nanoparticles in inducing M1 macrophage polarization in combination of exposure to ionizing radiations. Hence, in specific embodiments, the method of treating cancer further comprises exposing said subject to a radiotherapy, wherein the combined effect of (i) said nanoparticles containing metallic elements in inducing M1 macrophage polarization and (ii) said radiotherapy, produces a therapeutic effect on said cancer.

Radiation therapy or radiotherapy is the medical use of irradiation -i.e. ionizing radiation- as part of cancer treatment to control malignant cells. It is used as palliative treatment or as therapeutic treatment. Radiotherapy is accepted as an important standard therapy for treating various types of cancers.

As used herein, the term "radiotherapy" is used for the treatment of diseases of oncological nature with irradiation corresponding to ionizing radiation. Ionizing radiation deposits energy that injures or destroys cells in the area being treated (the target tissue) by damaging their genetic material, making it impossible for these cells to continue to grow.

In specific embodiment, the method of the disclosure comprises exposing the tumor to be treated to an efficient dose of ionizing radiations, wherein said ionizing radiations are photons, e.g. X-rays. Depending on the amount of energy they possess, the rays can be used to destroy cancer cells on the surface of or deeper in the body. The higher the energy of the X-ray beam, the deeper the X-rays can go into the target tissue. Linear accelerators and betatrons produce X-rays of increasingly greater energy. The use of machines to focus radiation (such as X-rays) on a cancer site is called external beam radiotherapy.

In an alternative embodiment of the method of treatment according to the disclosure, gamma rays are used. Gamma rays are produced spontaneously as certain elements (such as radium, uranium, and cobalt 60) release radiation as they decompose, or decay.

Ionizing radiations are typically of 2keV to 25000 keV, in particular of 2 keV to 6000 keV (i.e. 6 MeV) or of 2 keV to 1500 keV (such as cobalt 60 source).

A person of ordinary skill in the radiotherapy art knows how to determine an appropriate dosing and application schedule, depending on the nature of the disease and the constitution of the patient. In particular, the person knows how to assess dose-limiting toxicity (DLT) and how to determine the maximum tolerated dose (MTD) accordingly.

The amount of radiation used in radiation therapy is measured in gray (Gy), and varies depending on the type and stage of cancer being treated. For curative cases, the typical dose for a solid tumor ranges from 20 to 120 Gy. Many other factors are considered by radiation oncologists when selecting a dose, including whether the patient is receiving chemotherapy, patient co-morbidities, whether radiation therapy is being administered before or after surgery, and the degree of success of surgery.

The total dose is typically fractionated (spread out over time). Amount and schedules (planning and delivery of ionizing radiations, fraction dose, fraction delivery schema, total dose alone or in combination with other anti-cancer agents etc) is defined for any disease/anatomical site/disease stage patient setting/age and constitutes the standard of care for any specific situation.

A typical conventional fractionation schedule for adults may be 1.8 to 2 Gy per day, five days a week, for example for 5 to 8 consecutive weeks. In specific embodiments, said radiotherapy consists of exposing the subject to a total dose of ionizing radiations between 25 and 35 Gy, for example 30 Gy.

In other specific embodiments, the subject is exposed to a dose of ionizing radiations per fraction of about 3 to 8 Gy, and the total dose is administered preferably in a maximum of 10 fractions.

In certain specific embodiments, considering the synergistic effect obtained with the effect of inducing M1 macrophage polarization, a suboptimal total dose of ionizing radiations may be used in combination with the nanoparticles containing said high-Z element. As used herein, the term "suboptimal" refers to a total dose which would not be deemed sufficient according to the practice in the art, without induction of M2-polarized macrophage.

In a specific embodiment of the method of the present disclosure, said radiotherapy further comprises administering a radiosensitzing amount of high-Z element containing nanoparticles, which may be the same or different as nanoparticles containing the metallic elements previously administered for inducing M1 macrophage polarization, and exposing said subject to an efficient dose of ionizing radiations for radiotherapy, wherein the combined effect of (i) said nanoparticles containing metallic elements in inducing M1 macrophage polarization, (ii) said high-Z element containing nanoparticles as radiosensitizing agent, and (iii) the ionizing radiations, produces a therapeutic effect on said cancer.

For example, a radiosensitizing amount of high-Z element containing nanoparticles may be comprised between 15mg/kg and 200mg/kg per injection.

The method of the present disclosure for treating cancer comprises a step of administering an efficient amount of a suspension of the nanoparticles to the tumor of the subject.

The nanoparticles can be administered to the subject using different possible routes such as local (intra-tumoral (IT), intra-arterial (IA)), subcutaneous, intravenous (IV), intradermic, airways (inhalation), intra-peritoneal, intramuscular, intra-thecal, intraocular or oral route.

In specific embodiments, the nanoparticles are administered intravenously, and the nanoparticles are advantageously targeted to the tumors, by passive targeting, for example by enhanced permeability and retention effect.

Repeated injections or administrations of nanoparticles can be performed, when appropriate.

In one embodiment, the nanoparticles is administered to the patient an amount so that, after at least 24 hours following the administration of the nanoparticles, the metallic element (e.g. gadolinium or bismuth) concentration in the tumor, is between 0,1 and 10 g metallic element.g-1.

In a specific embodiment, a single dose between 15 mg/kg and 200 mg/kg of nanoparticles, for example, between 50 mg/kg and 150 mg/kg (for example the ultrafine nanoparticles with chelates of gadolinium) is injected intravenously in a subject.

In a specific embodiment, the nanoparticles are administered to the tumor of the patient so that, the nanoparticles are present in the region of the tumor to be treated at a concentration between 0,1mg/l and 1g/l, preferably between 0,1 and 100mg/l.

Other aspects and advantages of the method of the disclosure will become apparent in the following examples, which are given for purposes of illustration only.

### Examples

### Materials and Methods

### Cells, reagents and Gadolinium-based nanoparticles

The human monocyte cell line THP1 cells were maintained in RMPI-1640-Glutamax medium (Life technology) supplemented with 10% heat-inactivated fetal bovine serum (Hycultec GmbH) and 100 IU/ml penicillin-streptomycin (Life technology). To obtain THP1 macrophages, THP1 monocytes were differentiated with 320 nM of Phorbol 12-myristate 13-acetate (PMA) (#tlrl-PMA, Invivogen) during 24 hours. Gadolinium-based Nanoparticle (GdBN, AGuIX) were synthesized as described hereafter and resuspended in Hank's balanced salt solution (HBSS, Gibco).

AGuIX are sub-6 nm nanoparticles that are composed of a polysiloxane matrix with gadolinium cyclic chelates covalently grafted on the inorganic matrix. These nanoparticles have demonstrated very high radiosensitizing effects with excellent MRI positive contrast properties due to the presence of gadolinium.

### Preparation of gadolinium oxide cores.

A solution was prepared by dissolving 167.3 g of [GdCl₃, 6 H₂O] in 3L of DEG at room temperature. The solution was then stirred for 3 hours at 140°C. 44.5 mL of sodium hydroxide solution (10 M) are then added to the precedent solution and the solution is stirred for 5 hours at 180°C before cooling and stirring for 12 hours at ambient temperature. The gadolinium oxide cores displayed hydrodynamic diameters of 1.7 ± 0.5 nm.

### Encapsulation of gadolinium oxide cores by polysiloxane.

The polysiloxane shell is ensured by sol-gel process by addition of silane precursors. First, a solution containing 1.6 L of DEG, 51.42 mL of TEOS and 80.61 mL of APTES is slowly added (during 96 hours) to the precedent solution under stirring at 40°C. One hour after the end of the addition of the silane precursors, a second solution of 190 mL of DEG, 43.1 mL of water and 6.94 mL of TEA is added under stirring at 40°C during 96 hours. At the end of the second addition, the solution is stirred for 72 hours at 40°C and finally for 12 hours at ambient temperature. The gadolinium oxide cores coated by the polysiloxane displayed hydrodynamic diameter of 2.6 ± 1.0 nm.

### Covalent grafting of DOTAGA on the nanoparticles.

163.7 g of DOTAGA anhydride are then added to the core-shell nanoparticles in DEG. The resulting solution is then stirred for 72 hours at room temperature.

### Purification.

17.5 L of acetone are then added to the solution to precipitate the nanoparticles that are filtered under vacuum before dispersion in water. The solution is stirred for one hour at pH 2 before removing of remaining acetone by evaporation. The nanoparticles are then purified by tangential filtration though 5 kDa membrane. The solution is then stirred at pH 5 for 12 hours before another tangential filtration through 5 kDa membrane after addition of sodium hydroxide solution to reach pH 7.4. Then the solution was filtered twice through a 1.2 µm and then 0.2 µm syringe filters to remove the largest impurities. Finally, the nanoparticles are freeze-dried and can be stored for months without alterations. After dispersion in water, the AGuIX nanoparticles display hydrodynamic diameter of 2.2 ± 1 nm.. An average mass of 8.7 ± 1 kDa is obtained by mass spectrometry. The longitudinal relaxivity (r₁) is 11.5 mM⁻¹.s⁻¹ at 1.4 T. The nanoparticles display an isoelectric point at pH 7.5 ± 0.5. About 50 g of nanoparticles are obtained at the end of the synthesis.

### Antibodies

Antibodies used for immunofluorescence were anti-iNOS (#3523) antibodies from Abcam and anti-phospho-H2AX (Ser139) (#05-636) antibody from EMD Millipore. Antibodies used for immunoblots were anti-phospho-ATM (Ser1981) (10H11.E12) (#4526) and anti-ATM (D2E2) (#2873) antibodies from Cell Signaling Technology; anti-IRF5 (#ab21689), antibodies were from Abcam; anti-beta Actin antibody (AC-15) (HRP) (#49900,Abcam) was used as a loading control.

### Irradiation

Cells were seeded in 6-well plates, 12-well plates or 25 cm² flasks and irradiated with X-ray irradiator (1 Gy/min, X-RAD 320, Precision X-Ray). Cells were harvested at indicated time points after irradiation for subsequent experiments.

### Immunofluorescence microscopy

Cells were grown on coverslips and were treated as indicated. After treatment, cells were rinsed twice, fixed with 10% neutral buffered formalin (Sigma-Aldrich) for 10 minutes and then permeabilized with 0.3% Triton X-100 in PBS for 15 minutes. Cells were then washed twice with PBS and were blocked with 10% FBS in PBS for 1 h at room temperature, followed by incubation with primary antibodies in 10% FBS in PBS for 1 h 30 at room temperature. Then, samples were incubated with secondary antibodies using Alexa Fluor-488 green or Alexa Fluor-546 red (Life Technologies) and Hoechst 33342 for nuclei (Thermo Fisher Scientific) in 10% FBS in PBS for 30 minutes at room temperature. Coverslips were mounted with Fluoromount-G (SouthernBiotech) ant then visualized with Leica TCS SPE confocal microscope (Leica Microsystemes, France) using a 63X objective.

### Immunoblots

Cells were washed twice with cold PBS and lysed with NEHN buffer (0.5% NP40, 20% Glycerol, 300 mM NaCl, 20 mM Herps, pH 7.5, and 1 mM EDTA) complemented with 2.5 mM DTT and the protease and phosphatase inhibitor (Roche) at 4 °C. 5-20 g of proteins were separated by NuPAGE 4-12% or 10% SDS-PAGE gel (Invitrogen) and then were transferred onto a nitrocellulose membrane (0.2 Micron, Bio-Rad). Membranes were blocked with 5% nonfat milk or 5% Bovine Serum Albumine (BSA) in Tris-buffered saline and 0.1% Tween 20 (TBS-T) at room temperature for 1 hour and then subsequently probed with primary antibodies overnight at 4 °C. Then, membranes were incubated with appropriate horseradish peroxidase-conjugated anti-rabbit or anti-mouse IgG (SouthernBiotech) for 1 hour at room temperature. After 3 washes with TBS-T, immunoblots were revealed using G:BOX Chemi XL1.4 Fluorescent & Chemiluminescent Imaging System (Syngene).

### Statistical analysis

ANOVA test was used to establish statistical differences in biological activity (iNOS expression) and in phosphorylation (-H2AX) of macrophages Statistically significant values are reported in figure legends. All experiments were independently performed at least three times. Data are expressed as mean ± SEM. GraphPad Prism version 6.0b (GraphPad Software) was employed to perform statistical analysis.

### RESULTS

### Gadolinium-based nanoparticles and their combination with ionizing radiation trigger DNA damage and a DNA damage response in anti-inflammatory human macrophages.

To study the biological effects of GdBN alone and the combination of GdBN with XR (GdBN+XR) in anti-inflammatory macrophages, we first determined the ability of these treatments to induce DNA damage and ATM-dependent DNA damage response that we previously involved in IR-mediated macrophage reprogramming (Wu et al., Cell death Diff (2017)). By using fluorescent microscopy, we explored the DNA damage (as revealed by the detection of -H2AX foci) in PMA-treated human THP1 monocytes exposed to 0.2 Grays of X-rays and/or to different concentrations of GdBN. As previously *reported (*Wu et al., Cell Death Differ. 24(2017):1632-16), we observed after 30 minutes that XR alone induced the phosphorylation of H2AX on serine 139 (H2AXS139*) on macrophage nuclei, also known as nuclear -H2AX⁺ foci (Figures 1A and 1C). In addition, we also revealed that the combination of 200 nM GdBN with XR significantly enhanced the ability of XR to induce nuclear accumulation of - H2AX⁺ foci on macrophages, thus confirming our prediction that the combination GdBN+XR increases the dose deposition on macrophages and may thus induce pro-inflammatory macrophage reprogramming. Moreover, we detected after 2 hours that PMA-treated human THP1 monocytes that were treated with 200 nM GdBN also exhibited -H2AX foci (Figures 1B and 1C), indicating that GdBN alone may also directly trigger DNA damage in human macrophage. Interestingly, we noticed that the DNA damage could not be detected after 48 hours (Figure 1C). We then studied the activation of a DNA damage response (DDR) in treated PMA-treated human THP1 monocytes by analyzing the activating phosphorylation of the kinase ATM on serine 1981 (ATMS1981*) by immunoblots and detected after 30 minutes, that GdBN alone induced ATMS1981* (Figure 1D) and that the level of ATMS1981* phosphorylation is significantly increased after GdBN+XR combination, as compared to control cells (Figure 1D). Altogether, these results revealed that the ability of GdBN to *(i)* directly induce DNA damage on anti-inflammatory macrophages and *(ii)* to enhance the amount of DNA damages that are detected after the irradiation of anti-inflammatory macrophages.

### Gadolinium-based nanoparticles and their combination with ionizing radiation favor the reprogramming of anti-inflammatory macrophages into pro-inflammatory macrophages.

We then studied the impact of GdBN alone and GdBN+XR combination on macrophage functional reprogramming and determined by immunoblot the expression the IFN regulatory factor 5 (IRF5), a central transcription factor that has been involved in the pro-inflammatory macrophage reprogramming (Krausgruber et al., Nat Immunol. 12(2011):231-8). We observed after 48 hours of irradiation that the combination of XR with 100 nM or 200 nM GdBN enhanced the expression of IRF5 (Figure 2A). Interestingly, we also detected that 200 nM GdBN alone increased the expression of IRF5 in PMA-treated human THP1 monocytes (Figure 2A), indicating that GdBN alone and GdBN+XR combination induce pro-inflammatory macrophage reprogramming. To confirm these results, we also determined using fluorescent microscopy the expression level of another pro-inflammatory marker of macrophage activation, the inducible nitric oxide synthase (iNOS) on PMA-treated human THP1 monocytes that were irradiated with 0.2 Gy in combination or not with 100 or 200 nM GdBN. Although PMA-treated human THP1 monocytes that were treated with 100 nM or 200 nM of GdBN and/or irradiated with 0.2 Gy did not exhibit an increased expression of iNOS 2 hours post-irradiation, the frequency of iNOS⁺ macrophages significantly increased after 48 hours in presence of 100 nM or 200 nM GdBN alone, 0.2 Gy irradiation or the combination of 200 nM GdBN with 0.2 Gy irradiation (Figures 2B and 2C). Altogether, these results indicate GdBN alone and the combination of GdBN with XR can promote a cell-autonomous activation of human macrophages toward a pro-inflammatory phenotype.

## Claims

1. Nanoparticles containing metallic elements, for use in a method of treating a condition associated with undesirable M2-polarized macrophages in a subject in need thereof, the method comprising administering said nanoparticles containing metallic elements in said subject in need thereof;
wherein said nanoparticles have a mean hydrodynamic diameter below 10 nm, for example between 1 and 8 nm, preferably between 2 and 6 nm.

2. The nanoparticles for use in a method of Claim 1, wherein said nanoparticles containing metallic elements are administered to said subject in an amount sufficient for in vivo inducing M1 macrophage polarization.

3. The nanoparticles for use in a method of Claim 1 or 2, wherein said metallic elements are cationic elements, either as oxide and/or chalcogenide or halide or as a complex with a chelating agent, such as an organic chelating agent.

4. The nanoparticles for use in a method of any one of Claims 1-3, wherein said metallic elements are selected among the following elements: titanium, vanadium, chromium, manganese, iron, cobalt, nickel, copper, zinc, zirconium, niobium, molybdenum, hafnium, tantalum, tungsten, lanthanum, yttrium, and any other lanthanides.

5. The nanoparticles for use in a method of any one of Claims 1-4, wherein said metallic elements are selected among those suitable for use as T1 contrast agent in magnetic resonance imaging (MRI), for example selected from the list consisting of: manganese, gadolinium, europium and terbium.

6. The nanoparticles for use in a method of any of Claims 1-5, wherein said metallic elements are further selected among high-Z elements, wherein said high-Z element is an element with an atomic Z number higher than 40, preferably higher than 50.

7. The nanoparticles for use in a method of any one of Claims 1-6, wherein said subject is not exposed to ionizing radiations for radiotherapy within a period of at least 7 days, for example at least 10 days, following the administration of said nanoparticles containing metallic elements.

8. The nanoparticles for use of in a method of any one of Claims 1-7, wherein said condition associated with undesirable M2-polarized macrophages is cancer, preferably cancer of head and neck, skin cancer, liver cancer, colon cancer, pancreatic cancer, breast cancer, ovary cancer, uterus cancer, cervical cancer, brain cancer, thyroid cancer, bladder cancer and renal cancer.

9. The nanoparticles for use in a method of any of Claims 1-8, wherein said method further comprises administering to said subject an immunotherapeutic treatment in combination with said metallic element containing nanoparticles, wherein the combined effect of (i) said metallic element containing nanoparticles in inducing M1 macrophage polarization and (ii) the immunotherapeutic treatment, produces a therapeutic effect on said cancer.

10. The nanoparticles for use in a method of Claim 9, wherein said immunotherapeutic treatment comprises administering a therapeutically efficient amount of one or more immunotherapeutic drugs selected from the group consisting of anti-PD1 or anti-PDL1 antibodies, (e.g. nivolumab, pembrolizumab, avelumab, durvalumab, cemiplimab, durvalumab or atezolizumab) or anti-CTLA-4 antibodies (e.g. ipilimumab).

11. The nanoparticles for use of in a method of any one of Claims 1-10, wherein said method further comprises exposing said subject to a radiotherapy, wherein the combined effect of (i) said metallic element containing nanoparticles in inducing M1 macrophage polarization and (ii) said radiotherapy, produces a therapeutic effect on said cancer.

12. The nanoparticles for use in a method of any one of Claims 1-11, wherein a condition associated with undesirable M2-polarized macrophages is an inflammatory disorder, including progressive fibrotic diseaases, such as for example idiopathic pulmonary fibrosis, hepatic fibrosis systemic sclerosis, allergy, asthma and atherosclerosis.

13. The nanoparticles for use of any one of Claims 1-12, wherein said nanoparticles comprise
• polyorganosiloxanes with a silicium weight ratio of at least 8% of the total weight of the nanoparticle, preferably between 8% and 50%,
• chelates covalently bound to said polyorganosiloxane, in a proportion comprising between 5 and 100, preferably between 5 and 20 per nanoparticle, and,
• metallic elements complexed to the chelates.

14. The nanoparticles for use of any one of Claims 1-13, wherein said nanoparticles comprises chelates for complexing the metallic elements, obtained by grafting one or more of the following chelating agents on said nanoparticles: DOTA, DTPA, EDTA, EGTA, BAPTA, NOTA, DOTAGA, and DTPABA, or their mixtures.

15. The nanoparticles for use of any one of Claims 1-14, wherein said nanoparticles are gadolinium-chelated polysiloxane nanoparticles of the following formula 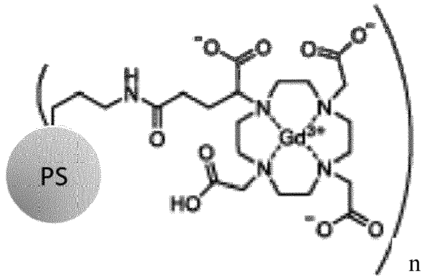 wherein PS is a matrix of polysiloxane and n is comprised between 5 and 50, and wherein the hydrodynamic diameter is comprised between 1 and 8 nm, typically between 2 and 6 nm.
